# FASCICULE DE BREVET EUROPEEN

(11) **EP 0 967 967 B1**
(45) Date de publication et mention de la délivrance du brevet: **10.07.2002**
(21) Numéro de dépôt: 97906237.9
(22) Date de dépôt: 20.02.1997
(51) Int. Cl.: A61K 7/48, A61K 7/06, A61K 31/045, A61K 9/00

(54) **COMPOSITIONS COSMETIQUES, DERMOPHARMACEUTIQUES OU VETERINAIRES POUR LE TRAITEMENT ASEPTISANT DE LA PEAU HUMAINE OU ANIMALE**
KOSMETISCHE, DERMOPHARMAZEUTISCHE ODER TIERÄRZTLICHE ZUSAMMENSETZUNGEN ZUR ANTISEPTISCHEN BEHANDLUNG VON MENSCHLICHER ODER TIERISCHER HAUT
COSMETIC, DERMOPHARMACEUTICAL OR VETERINARY COMPOSITIONS FOR DISINFECTING HUMAN OR ANIMAL SKIN

(30) Priorité: 21.02.1996 FR 9602313; 23.04.1996 FR 9605210
(43) Date de publication de la demande: 05.01.2000
(73) Titulaire: Stoa S.A., 78610 Le Perray-en-Yvelines Cedex (FR)
(72) Inventeur: GREFF, Daniel, F-78490 Mère (FR)
(74) Mandataire: Laget, Jean-Loup
(86) Numéro de dépôt international: FR9700320
(87) Numéro de publication internationale: WO9730692

(56) Documents cités:
- EP-A- 0 025 640
- EP-A- 0 336 803
- EP-A- 0 524 548
- EP-A- 0 584 692
- EP-A- 0 623 336
- WO-A-92/19216
- WO-A-93/24101
- WO-A-94/21234
- DE-A- 4 320 744
- FR-A- 2 125 530
- FR-A- 2 678 830
- FR-A- 2 682 296
- US-A- 4 049 830
- US-A- 5 190 978
- PATENT ABSTRACTS OF JAPAN vol. 005, no. 067 (C-053), 7 Mai 1981 & JP 56 018910 A (KANEBO LTD), 23 Février 1981,
- DATABASE WPI Section Ch, Week 9039 Derwent Publications Ltd., London, GB; Class A97, AN 90-294123 XP002022305 & JP 02 206 695 A (KAO CORP) , 16 Août 1990
- DATABASE WPI Section Ch, Week 7639 Derwent Publications Ltd., London, GB; Class C03, AN 76-72925X XP002022306 & JP 51 091 327 A (KAO SOAP KK) , 11 Août 1976

## Description

La peau humaine ou animale (mammifères) n'est pas stérile. Elle héberge soit des germes saprophytes qui constituent la microflore résidente non pathogène et protectrice, soit des germes pathogènes parasites, opportunistes, qui peuvent engendrer des maladies ou des états pathologiques plus ou moins graves, surtout s'ils réussissent à pénétrer à l'intérieur du tissu pour coloniser le système sanguin, lymphatique ou des organes internes.

Le traitement aseptisant des peaux humaines et/ou animales cherche ses origines dans les travaux de Pasteur et de Semmelweiß; aujourd'hui, il existe un grand nombre de molécules microbicides utilisées pour différentes applications: traitement de l'acné, traitement des pellicules capillaires, désinfection des petites plaies (égratignures), désinfection des mains des chirurgiens et infirmières, désinfection des pis des mamelles des bêtes laitières pour prévenir les mammites.

Les propriétés anti-microbiennes des alcanes 1,2-diol et alcanes 1,3-diol sont connues de EP-A-0524548 qui mentionne également leur combinaison avec un alcool aromatique pour la conservation d'agent nettoyants de la peau, mais avec une diminution des germes seulement après plusieurs jours.

L'effet bactériostatique des composés de formule R-CHOH-CH₂OH sur les bactéries gram + et les moisissures, est connu de JP-A-51 091 327 en vue d'une application sur les produits alimentaires et les semences, ou autres produits industriels.

L'objet de la présente demande de brevet est la découverte que la classe de molécules de formule générale:

R-CHOH-CH₂-OH (I)

où R est une chaîne alkyle linéaire ou ramifiée de 3 à 12 atomes de carbone, préférentiellement linéaire de 6 à 8 atomes de carbone, possède une activité microbicide suffisamment forte pour être utilisable dans les traitements de la peau évoqués précédemment. Ces molécules alcane-diols ont été décrites en tant qu'ingrédients hydratants, en tant que conservateurs antimicrobiens pour la protection des produits cosmétiques; leur application topique sur la peau pour combattre les microbes pathogènes n'a pas été envisagée.

De plus, il a été découvert que la combinaison des alcane-diols avec des gels de type glycéryl poly(méth)acrylate dans une formule est encore plus efficace que chacun des composants seuls.

Le brevet FR 2682296 a proposé une méthode de conservation non chimique de produits cosmétiques ou dermopharmaceutiques qui est basée sur l'utilisation de gels de type glycéryl poly(méth)acrylate, dont la propriété est d'exercer un fort effet osmotique sur leur environnement, ce qui permet d'inactiver les micro-organismes introduits dans une préparation cosmétique en les privant d'eau. Le brevet FR 2.737.406 a proposé une amélioration de l'efficacité de la méthode en associant des polyols et un agent fluidifiant au gel poly(méth)acrylate.

DE 43 20 744 A décrit l'utilisation des 1,2 alcane-diols pour réguler l'humidité de la peau dans des compositions cosmétiques.

EP-A-0 524 548 ou EP-A-0 584 692 ou FR-A-2 682 296 décrit l'utilisation des alcane-diols pour la conservation des produits cosmétiques nettoyants de la peau ou des produits alimentaires.

US-A-5 190 978 décrit des compositions comprenant des alcane-diols pour le traitement du cancer.

DATABASE WPI Section Ch, Week 7639 Derwent Publications Ltd., London, GB; Class C03, AN 76-72925X XP002022306 & JP 51 091 327 A décrit l'utilisation des gels de type glyceryl poly(meth)acrylate pour la conservation des produits cosmétiques et éviter l'addition des conservateurs antimicrobiens (revendication 1 et pages 3-4).

US-A-4 049 830 décrit une composition pour le traitement de mammites qui ne contient pas d'alcane-diols.

L'objet de la présente demande de brevet est que les alcane-diols peuvent être utilisés en combinaison avec les gels cités à des faibles concentrations pour le traitement des problèmes d'acné, de pellicules, d'impétigo, des odeurs corporelles causées par les micro-organismes, des causes du "pied d'athlète" et d'autres mycoses, des affections microbiennes cutanées en général, et la prévention des mammites par application topique.

Un gel de glycéryl poly(méth)acrylate comme ceux décrits dans les brevets, FR 2682296 ou FR 2678830 qui inactive les micro - organismes introduits dans une préparation cosmétique en les privant d'eau, perd son activité quand il est trop dilué. Cependant, lorsqu'il est additionné d'un alcane-diol de formule (I), même à faible concentration, on constate un effet d'inactivation des germes, rapide et beaucoup plus fort que l'addition des deux substances ne laisse supposer.Il y a donc synergie entre le gel, avec son pouvoir osmotique, et l'alcane-diol, et son effet antimicrobien.
Ceci est d'autant plus important qu'il permet de diminuer les concentrations de diol nécessaires pour obtenir un effet microbicide dans les produits finis. Les alcanediols de courte chaîne peuvent présenter des propriétés légèrement irritantes, la concentration utilisable sans risque est donc limitée. La synergie avec le gel permet de surmonter cet obstacle.

Le gel de type glycéryl polyacrylate ou polyméthacrylate est particulièrement important pour réaliser cette synergie. Il se compose de glycérine (variant entre 20 et 90%, préférentiellement entre 50 et 75%), d'eau (variant entre 10 et 80%, préférentiellement entre 20 et 49%) et de polyacrylate et/ou -méthacrylate (variait entre 0,1 et 5%) et présente une viscosité comprise entre 50.000 et 2.000.000 centipoises. Ce gel clathrate se caractérise par son fort pouvoir de rétention d'eau. Il ne sèche pas, même exposé pendant des mois à l'air ambiant ou soumis au vide d'air pendant 48 heures. Cette propriété est essentielle, les hydrogels classiques, les gels d'alginate, de polysaccharide, de cellulose ou ses dérivés ou de silicate ne satisfont pas ce critère. Ce gel est particulièrement efficace pour inactiver les micro-organismes avec lesquels il entre en contact, privant les germes par son effet osmotique de l'eau dont ils ont besoin pour survivre.

La concentration en gel glycéryl poly(méth)acrylate peut varier entre 1 et 99 %, préférentiellement entre 5 et 20 %.

Ce poly(méth)acrylate est choisi de préférence parmi les sels sodiques, potassiques, triéthylaminiques, triéthanolaminiques, ammoniacaux, de l'acide acrylique et/ou de l'acide méthacrylique, mais aussi parmi les esters ou les amides de ces polymères acides, ou les dérivés réticulés du type carbomère (réticulation par des éthers allyliques de pentaérythritol, de sucrose ou de propylène, par exemple).
Dans les compositions cosmétiques, dermopharmaceutiques ou vétérinaires selon l'invention, la concentration de l'alcane-diol varie entre 0,01 et 5 %
(p/p), préférentiellement entre 0,1 et 1,0 % (p/p) dans le produit fini.

De manière avantageuse, l'alcane-diol est l'octane - 1,2 diol.

Les compositions de la présente demande de brevet peuvent être utilisées dans toute forme galénique employée en cosmétique, dermopharmacie ou en application vétérinaire par voie topique à vocation aseptisante ou anti-microbienne: solutions aqueuses, émulsions H/E et E/H, laits, lotions, gels, pommades, lotions capillaires, shampooings et après-shampooings, savons, sticks, sprays, cataplasmes, pansements, sans que cette liste soit limitative.

Les compositions selon l'invention peuvent en outre contenir au moins un solvant fluidifiant de formule générale:

R₁-O-(R₂-0-R₂)ₙ-OR₃ (II)

dans laquelle R₁ est un atome d'hydrogène ou une chaîne alkyle en C1 à C5 linéaire ou ramifiée, R₂ est une chaîne alkyle en C1 à C5 linéaire ou ramifiée, R₃ est un atome d'hydrogène ou une chaîne alkyle en C1 à C5 linéaire ou ramifiée et n est une valeur entière de 1 à 200,000.

De préférence, R₁ est une chaîne alkyle en C1 à C3 linéaire et n est une valeur entre 1 et 3.

La proportion du solvant fluidifiant est avantageusement comprise entre 0,5 à 50 %, et de manière préférée varie entre 1 à 10%.

Les compositions selon l'invention renfermant également le solvant fluidifiant précité peuvent être utilisées pures, ou incorporées dans d'autres gels de base (carbomères, comme par exemple le Carbopol® de la Société Goodrich, ou gels polysaccharidiques, alginates, gommes xanthanes etc ...) à des concentrations variant de 3 à 50 %, préférentiellement entre 10 et 20 %, pour le traitement par trempage ou massage des pis de vaches ou d'autres animaux laitiers.
Les compositions contenant ces gels ont les avantages suivants: elles sont à priori incolores, elles sont thioxotropes, c'est-à-dire que le gel se fluidifie au contact avec la peau tout en laissant un film hydrique sur le pis lors du trempage; elles ne contiennent pas de conservateur ou désinfectant chimique, elles sont non-toxiques, non-irritantes, elles sont fortement hydratantes et adoucissantes pour la peau, et surtout elles possèdent une forte activité anti-microbienne contre les germes trouvés dans l'environnement de la ferme: Staphylococcus aureus, Streptococcus faecalis, Pseudomonas aeruginosa, Escherichia coli, Lysteria monocytogènes et nombreuses autres souches. Cette activité est due au pouvoir osmotique du gel, donc à une activité physique, et de sa combinaison avec le(s) polyol(s).

L'invention va être illustrée par les exemples suivants, non limitatifs:

### Exemple 1: Effet de synergie

### . alcane-diol seul:

1 ml d'une suspension de Staphylococcus Aureus (75,0.10⁶ germes/ml) est inoculée dans 10 g d'une solution d'octane-1-,2-diol dosé à 0,2 % dans l'eau).

Après 30 secondes, 15 minutes et 30 minutes de contact, on compte le nombre de micro-organismes revivifiables (étalement en gélose).

On obtient: à 30 secondes: environ 8,0.10⁶ germes/ml, à 15 minutes: environ 1,2.10⁶ germes/ml, à 30 minutes: environ 250.000 germes/ml, donc on constate un effet bactéricide à 30 minutes.

### . gel glycéryl polyméthacrylate seul (à 40 %)

Dans un protocole identique au précédent, on retrouve la totalité des germes inoculés après 30 secondes, 15 minutes et 30 minutes.

Inoculation avec S. Aureus: 3,0.10⁶ germes/g.: après 30 secondes, on retrouve 3,1.10⁶ germes/g., après 15 minutes 3,6.10⁶ germes/g, et après 30 minutes 3,9.10⁶ germes/g.

A 40 % dans l'eau, ce gel n'inactive plus les micro-organismes testés.

### . gel + alcane-diol:

Un mélange de gel à 40 % dans l'eau et d'octane-1,2 diol dosé à 0.2 % conduit aux résultats suivants:

Inoculation avec S. Aureus: 5,0.10⁶ germes/g: après 30 secondes, on retrouve 2,0. 10⁴ germes/g, après 15 minutes aucun germe revivifiable et après 30 minutes aucun germe revivifiable.

L'effet d'inactivation des germes est donc un effet synergique entre le gel et son pouvoir osmotique et l'alcane-diol, puisqu'il est beaucoup plus fort que l'addition des deux substances ne le laissait supposer.

### Exemple 2

Inactivation des germes responsables de l'acné Propionibacterium acnes: 10 g d'un gel de carbomère contenant 0.5% d'hexane-1,2-diol sont inoculés avec 4,8. 10⁶ germes/g d'une culture anaérobie à 35°C. Au temps t=0. t=15 minutes et t=30 minutes on prélève pour un comptage des germes revivifiables.

On ne retrouve aucun germe vivant, même au temps t=0 (maximum 30 secondes), alors que dans le gel sans hexane-1,2-diol, les germes prolifèrent (on retrouve entre 3,0 et 8,0. 10⁶ germes/g.

Un résultat identique est obtenu pour l'octane-1,2-diol contre la levure Pityrosporum ovale = Malassezia furfur inactivation rapide (en 15 minutes, la réduction logarithmique est d'un facteur 5).

L'application topique d'un produit contenant un alcane-1,2-diol selon l'invention a été testée dans les conditions suivantes:

10 personnes volontaires présentant des symptômes d'acné juvénile ont utilisé une solution aqueuse contenant 1% d'octane-1,2-diol pendant une période de 3 semaines. L'étude clinique avant et après le traitement a montré une amélioration sensible de l'état de la peau sur les sites traités, en comparaison avec les sites non-traités: diminution de la flore bactérienne, moins de pustules, papules, comédons ; peau moins grasse, plus lisse.

Une autre étude a été menée dans le milieu fermier: 20 vaches d'un troupeau de 40 vaches laitières ont été traitées biquotidiennement après la traite par le trempage des pis dans une solution aqueuse contenant 1% de décane-1,2-diol, 3% de glycérine, 2% de polysorbate 20 (Tween®20) et excipient qsp 100%. Après deux mois de traitement, le taux de mammites subcliniques et le taux de leucocytes dans le lait ont diminué significativement chez les vaches traitées en comparaison avec l'autre moitié du troupeau non traité.

L'effet protecteur antimicrobien des alcane-1-,2diols par application topique a été ainsi démontré dans des applications nouvelles.

Les compositions selon la présente invention peuvent être combinées avec tout autre ingrédient habituellement utilisé dans ces domaines cosmétiques, dermopharmaceutique, ou vétérinaire respectifs; lipides d'extraction et ou de synthèse, polymères gélifiants et viscosants, tensioactifs et émulsifiants, principes actifs hydro- ou liposolubles, extraits de plantes, extraits tissulaires, extraits marins, polyols, adoucissants, autres agents antimicrobiens.

Pour les compositions destinées à la protection des mamelles contre les mammites, on cite les formules suivantes:

### Exemple N°3

| | |
|---|---|
| Carbopol (R) 934 (Goodrich) | 0.2 |
| Glycéryl polyacrylate | 5 |
| Glycéryl polyméthacrylate | 5 |
| Butylène glycol | 5 |
| Ethoxyéthanolacétate | 4 |
| Ethylhexyglycol | 1 |
| Eau | qsp 100 |
| triéthanolamine | qsp pH5 |

### Exemple n° 4

| | |
|---|---|
| Carboxy-méthyle cellulose | 0.5 |
| Glycéryl polyméthacrylate | 20 |
| Polyéthylène glycol PEG 400 | 5 |
| 1,2 Pentanediol | 1.5 |
| Sorbitol | 10 |
| Eau | qsp 100 |

### Exemple n° 5

| | |
|---|---|
| Carbopol (R) 940 (Goodrich) | 0.2 |
| Glycéryl polyacrylate | 15 |
| Ethoxydiglycol | 4 |
| octane 1,2 diol | 1 |
| Eau | qsp 100 |
| Soude (30 %) | qsp pH 5.5 |

Ces exemples ne sont pas limitatifs. Le gel de base peut être préparé à partir des substances habituellement utilisées pour faire des hydrogels acceptables dans le domaine agro-alimentaire ou pour une application topique (carraghénanes, gommes de xanthanes et autres polysaccharides; polyacrylates ou polyméthacrylates et leurs dérivés réticulés comme les carbomères, silicates d'aluminium sodés etc ...).

Ces compositions ont un fort pouvoir antimicrobien qui est basé soit sur au moins un alcane-1-,2-diol seul en concentration suffisante, soit sur la présence conjointe d'au moins un alcane diol et du gel glycéryl poly(méth)acrylate. Les germes pathogènes du type Lysteria, Staphylococces, Streptococces, Pseudomonas sont inactivés très rapidement. Les germes du type Listerai inocua, qui posent un problème de goût lorsqu'ils se retrouvent dans le lait, sont inactivés également. Grâce à la formation d'un film de gel sur la peau du pis, l'effet antibactérien est rémanent entre deux traites. En plus, les compositions ont un pouvoir hydratant très important, elles améliorent l'état de la peau diminuent les crevasses et gerçures ainsi que les irritations.

Ces compositions à base d'alcane-1,2-diol avec ou sans gel de type glycéryl poly(méth)acrylate peuvent être additionnées de toute substance utile ou avantageuse habituellement utilisée dans le domaine de la prévention des mammites par trempage, pulvérisation ou massage des pis: iode ou iodophores, chloramine T, peroxyde d'hydrogène, ou autres substances bactéricides, ainsi que toute substance émolliente et regraissante (paraffine, huiles synthétiques ou végétales), cicatrisante (allantoïne, bisabolol, beurre de karité par exemple), soignante (extraits de plantes). Cette énumération n'est pas limitative.

Les compositions selon la présente invention, à base d'alcane 1,2-diol avec ou sans gel de type glycéryl poly(méth)acrylate trouvent donc leur utilisation pour l'obtention d'hydrogels destinés aux traitements et aux soins de la peau, des cheveux, des ongles et du cuir chevelu, à savoir en particulier, le traitement anti-acné, anti-pellicules, anti-impétigo, déodorant, anti-mycose.

Additionnées d'un solvant fluidifiant, elles peuvent être également utilisées pour l'obtention d'hydrogels destinés au traitement et la prévention de mammites par application topique sur les pis et les mamelles des animaux laitiers, à la diminution du risque de transmission de Listéria au lait, et à l'amélioration de l'état de la peau du pis.

## Revendications

1. Compositions cosmétiques, dermopharmaceutiques ou vétérinaires pour applications topique sur la peau et destinées à l'utilisation locale pour leur effet anti-microbien, **caractérisées en ce qu'**elles contiennent la combinaison d'au moins un alcane-diol de formule générale
R-CHOH-CH₂-OH (I)
où R est une chaîne alkyle linéaire ou ramifiée de 3 à 12 atomes de carbone, préférentiellement linéaire de 6 à 8 atomes de carbone, et d'un gel de type glycéryl poly(méth)acrylate pour renforcer l'activité anti-microbienne par un effet osmotique et obtenir une synergie des deux mécanismes.

2. Compositions cosmétiques, dermopharmaceutiques ou vétérinaires selon la revendication 1, **caractérisées en ce que** la concentration de l'alcane-diol varie entre 0,01 et 5 % (p/p), préférentiellement entre 0,1 et 1 % (p/p).

3. Compositions cosmétiques, dermopharmaceutiques ou vétérinaires selon les revendications 1 à 2, **caractérisées en ce que** l'alcane-diol est l'octane-1,2 diol.

4. Compositions cosmétiques, dermopharmaceutiques ou vétérinaires selon les revendications 1 à 3, **caractérisées en ce que** la concentration du gel glycéryl poly(méth)acrylate varie entre 1 et 99 %, préférentiellement entre 5 et 20 %.

5. Compositions cosmétiques, dermopharmaceutiques ou vétérinaires selon les revendications 1 à 4, **caractérisées en ce que** le gel glycéryl poly(méth)acrylate est à base de polymères dérivés de 1 'acide acrylique et/ou méthacrylique se présentant sous forme de sels, d'esters ou d'amides de ces acides.

6. Compositions cosmétiques, dermopharmaceutiques ou vétérinaires selon l'une quelconque des revendications 1 à 5 **caractérisées en ce qu'**elles contiennent en outre au moins un solvant fluidifiant de formule générale:
R₁-O-(R₂-0-R₂)ₙ-OR₃ (II),
dans laquelle R₁ est un atome d'hydrogène ou une chaîne alkyle en C1 à C5 linéaire ou ramifiée, R₂ est une chaine alkyle en C1 à C5 linéaire ou ramifiée, R₃ est un atome d'hydrogène ou une chaîne alkyle en C1 à C5 linéaire ou ramifiée et n est une valeur entière de 1 à 200.000.

7. Compositions selon la revendication 6 **caractérisées en ce que** R₁ est une chaîne alkyle en C1 à C3 linéaire et n est une valeur entre 1 et 3.

8. Compositions selon l'une quelconque des revendications 6 e t 7 **caractérisées en ce que** la proportion du solvant fluidifiant est de 0,5 à 50 % et préférentiellement de 1 à 10%.

9. Compositions selon l'une quelconque des revendications 1 à 8, **caractérisées en ce qu'**elles sont formulées sous toute forme galénique employée en cosmétique, dermopharmacie ou en application vétérinaire par voie topique à vocation aseptisante ou anti-microbienne: solutions aqueuses, émulsions H/E et E/H, laits, lotions, gels, pommades, lotions capillaires, shampooings et après-shampooings, savons, sticks, sprays, masques, cataplasmes, pansements.

10. Compositions selon l'une quelconque des revendications 1 à 9, **caractérisées en ce qu'**elles sont susceptibles d'être combinées avec tout autre ingrédient habituellement utilisé dans les domaines cosmétiques, dermopharmaceutiques ou vétérinaires respectifs: lipides d'extraction et ou de synthèse, polymères gélifiants et viscosants, tensioactifs et émulsifiants, principes actifs hydro- ou liposolubles, extraits de plantes, extraits, tissulaires, extraits marins, polyols, adoucissants, autres agents antimicrobiens.

11. Compositions selon l'une quelconque des revendications 1 à 10, **caractérisées en ce qu'**elles sont additionnées de toute substance utile ou avantageuse habituellement utilisée dans le domaine de la prévention des mammites des animaux laitiers par trempage, pulvérisation ou massage des pis: iode ou iodophores, chloramine T, peroxyde d'hydrogène, ou autres substances bactéricides, ainsi que toute substance émolliente et regraissante (paraffine, huiles synthétiques ou végétales), cicatrisante (allantoïne, bisabolol, beurre de karité), soignante (extraits de plantes).

12. Utilisation des compositions selon les revendications 1 à 10 pour l'obtention d'hydrogels destinés aux traitements et aux soins de la peau, des cheveux, des ongles et du cuir chevelu, à savoir en particulier le traitement anti-acné, anti-pellicules, anti-impétigo, déodorant, anti-mycose.

13. Utilisation des compositions selon l'une quelconque des revendications 1 à 11 pour l'obtention d'hydrogels destinés au traitement et à la prévention de mammites par application topique sur les pis et les mamelles des animaux laitiers, à la diminution du risque de transmission de Listeria au lait, et pour l'amélioration de l'état de la peau du pis.

14. Utilisation d'alcane-diol de formule générale R-CHOH-CH₂-OH où R est une chaîne alkyle linéaire ou ramifiée de 3 à 12 atomes de carbone, préférentiellement linéaire de 6 à 8 atomes de carbone, pour l'obtention d'hydrogels sans gel de glycérylpoly(méth)acrylate, destinés aux traitements de la peau, des cheveux, des ongles et du cuir chevelu, à savoir en particulier le traitement anti-acné, anti-pellicules, anti-impétigo, déodorant, anti-mycose.

15. Utilisation d'alcane-diol de formule générale R-CHOH-CH₂-OH où R est une chaîne alkyle linéaire ou ramifiée de 3 à 12 atomes de carbone, préférentiellement linéaire de 6 à 8 atomes de carbone, pour l'obtention d'hydrogels à base d'alcane-diol sans gel de glycérylpoly(méth)acrylate, destinés aux traitements et à la prévention de mammites par application topique sur les pis et les mamelles des animaux laitiers, à la diminution du risque de transmission de Listeria au lait, et pour l'amélioration de l'état de la peau du pis.

## Patentansprüche

1. Kosmetische, dermopharmakologische oder veterinäre Zusammensetzungen für topische Anwendungen auf der Haut und für lokale Anwendungen aufgrund ihrer anti-mikrobiellen Wirkung, **dadurch gekennzeichnet, daß** sie in Kombination enthalten:
mindestens ein Alkandiol der allgemeinen Formel
R-CHOH-CH₂-OH
in der R eine gerade oder verzweigte Alkylkette mit 3 bis 12 Kohlenstoffatomen, vorzugsweise eine gerade Kette mit 6 bis 8 Kohlenstoffatomen ist, und ein Gel vom Typ Glyceryl-Poly-(Meth)-Acrylat enthält zur Verstärkung der anti-mikrobiellen Wirkung durch einen Osmoseeffekt, und um eine Synergie der beiden Wirkungsmechanismen zu erhalten.

2. Kosmetische, hautpharmakologische oder veterinäre Zusammensetzung nach Anspruch 1,
**dadurch gekennzeichnet, daß** die Konzentration des Alkandiols zwischen 0,01 und 5% (p/p), vorzugsweise zwischen 0,1 und 1% (p/p) variiert.

3. Kosmetische, hautpharmakologische oder veterinäre Zusammensetzung nach Anspruch 1 bis 2,
**dadurch gekennzeichnet, daß** das Alkandiol Oktan-1,2-Diol ist.

4. Kosmetische, hautpharmakologische oder veterinäre Zusammensetzung nach den Ansprüchen 1 bis 3,
**dadurch gekennzeichnet, daß** die Konzentration des Glyceryl-Poly-(Meth)-Acrylat-Gels zwischen 1 und 99%, vorzugsweise zwischen 5 und 20% variiert.

5. Kosmetische, hautpharmakologische oder veterinäre Zusammensetzung gemäß den Ansprüchen 1 bis 4,
**dadurch gekennzeichnet, daß** das Glyceryl-Poly-(Meth)-Acrylatgel auf von Acrylsäure und/oder Methacrylsäure abgeleiteten Polymeren basiert, die in Form von Salzen, Estern oder Amiden dieser Säuren vorliegen.

6. Kosmetische, hautpharmakologische oder veterinäre Zusammensetzungen nach einem der Ansprüche 1 bis 5,
**dadurch gekennzeichnet, daß** sie zusätzlich mindestens ein fluidisierendes Lösungsmittel der allgemeinen Formel
R₁-O-(R₂-0-R₂)ₙ-OR₃
enthalten, in der R₁ ein Wasserstoffatom oder eine gerade oder verzweigte Alkylkette mit C1 bis C5, R₂ eine gerade oder verzweigte Alkylkette mit C1 bis C5, R₃ ein Wasserstoffatom oder eine gerade oder verzweigte Alkylkette mit C1 bis C5 ist und n eine ganze Zahl von 1 bis 200 000 ist.

7. Zusammensetzungen nach Anspruch 6,
**dadurch gekennzeichnet, daß** R₁ eine gerade Alkylkette mit C1 bis C3 und n eine Zahl zwischen 1 und 3 ist.

8. Zusammensetzungen nach einem der Ansprüche 6 und 7,
**dadurch gekennzeichnet, daß** der Anteil des fluidisierenden Lösungsmittels von 0,5 bis 50% und vorzugsweise von 1 bis 10% beträgt.

9. Zusammensetzung nach einem der Ansprüche 1 bis 8,
**dadurch gekennzeichnet, daß** sie in beliebiger galenischer Form formuliert sind, die in der Kosmetik, Dermapharmakologie oder in Veterinäranwendung auftropischem Wege für sterile oder antimikrobielle Zwecke angewendet werden: wässrige Lösungen, Öl/Wasser- und Wasser/Öl-Emulsionen, Milch, Lotionen, Gele, Pomaden, Kapillarlotionen, Shampoos und After-Shampoos, Seifen, Stifte, Sprays, Masken, Umschläge, Verbände.

10. Zusammensetzungen nach einem der Ansprüche 1 bis 9,
**dadurch gekennzeichnet, daß** sie kombiniert werden können mit jedem anderen Ingredienz, das üblicherweise im Kosmetik-, Dermopharmakologie- oder Veterinärbereich verwendet werden:
extrahierte oder synthetische Lipide, gelbildende und zähflüssigmachende Polymere, Tenside und Emulgatoren, wasser- oder fettlösliche Wirkstoffe, Pflanzenextrakte, Gewebeextrakte, Meeresextrakte, Weichmacher-Polyole, andere antimikrobielle Wirkstoffe.

11. Zusammensetzung nach einem der Ansprüche 1 bis 10,
**dadurch gekennzeichnet, daß** ihnen jede beliebige nutz- oder vorteilbringende Substanz zugemischt ist, die üblicherweise zur Vorbeugung gegen Euterentzündung bei Milchtieren durch Eintunken, Besprühen oder Massage des Euters verwendet wird:
Jod oder Jodverbindungen, Chloramin T, Wasserstoffperoxyd oder andere bakterizide Substanzen, sowie jegliche Substanz zum Weichmachen und Rückfetten (Paraffine, synthetische Öle oder Pflanzenöle), zur Vernarbung (Allantoin, Bisabolol, Karitébutter), zur Pflege (Pflanzenextrakte).

12. Verwendung von Zusammensetzungen gemäß den Ansprüchen 1 bis 10, zur Gewinnung von Hydrogelen für die Behandlung und Pflege der Haut, der Haare, der Nägel und der Kopfhaut, insbesondere für die Behandlung gegen Akne, Schuppen, Hautausschlag, Körpergeruch, Hautpilz.

13. Verwendung von Zusammensetzungen gemäß den Ansprüchen 1 bis 11, zur Gewinnung von Hydrogelen für die Behandlung und Vorbeugung gegen Euterentzündung durch topisches Auftragen auf die Euter und die Brustdrüsen von Milchtieren, für die Verminderung des Risiko, Listeria auf Milch zu übertragen, und für die Verbesserung vom Euterhautzustand.

14. Verwendung von Alkan-diol der allgemeinen Formel R-CHOH-CH₂-OH in der R eine gerade oder verzweigte Alkylkette mit C₃ bis C₁₂, vorzugsweise eine gerade Alkylkette mit C₆ bis C₈, ist, zur Gewinnung von Hydrogelen ohne Glyceryl-Poly-(Meth)-Acrylatgel, für die Behandlung der Haut, der Haare, der Nägel und der Kopfhaut, insbesondere für die Behandlung gegen Akne, Schuppen, Hautausschlag, Körpergeruch, Hautpilz.

15. Verwendung von Alkan-diol der allgemeinen Formel R-CHOH-CH₂-OH in der R eine gerade oder verzweigte Alkylkette mit C₃ bis C₁₂, vorzugsweise eine gerade Alkylkette mit C₆ bis C₈, ist, zur Gewinnung von auf Alkan-diol basierten Hydrogelen ohne Glyceryl-Poly-(Meth)-Acrylatgel, für die Behandlung und Vorbeugung gegen Euterentzündung durch topisches Auftragen auf die Euter und die Brustdrüsen von Milchtieren, für die Verminderung des Risiko, Listeria auf Milch zu übertragen, und für die Verbesserung von Euterhautzustand.

## Claims

1. Cosmetic, medicated skin care or veterinary compositions for topical application to the skin and intended for local use for their antimicrobial action, **characterised in that** they contain a combination of at least one alkanediol of the general formula
R-CHOH-CH₂-OH (I)
wherein R is a linear or branched alkyl chain of 3 to 12 carbon atoms, preferably a linear chain of 6 to 8 carbon atoms, and of a gel of the glyceryl poly(meth)acrylate type for reinforcing the antimicrobial activity by an osmotic effect and achieving synergy between the two mechanisms.

2. Cosmetic, medicated skin care or veterinary compositions according to claim 1, **characterised in that** the concentration of alkanediol varies between 0.01 and 5 % (wt/wt), preferably between 0.1 and 1 % (wt/wt).

3. Cosmetic, medicated skin care or veterinary compositions according to claims 1 and 2, **characterised in that** the alkanediol is 1,2-octanediol.

4. Cosmetic, medicated skin care or veterinary compositions according to claims 1 to 3, **characterised in that** the concentration of glyceryl poly(meth)acrylate) gel varies between 1 and 99 %, preferably between 5 and 20 %.

5. Cosmetic, medicated skin care or veterinary compositions according to claims 1 to 4, **characterised in that** the glyceryl poly(meth)acrylate gel is based on polymers derived from acrylic and/or methacrylic acid in the form of salts, esters or amides of these acids.

6. Cosmetic, medicated skin care or veterinary compositions according to any one of claims 1 to 5, **characterised in that** they additionally contain at least one liquefying solvent of the general formula:
R₁-O-(R₂-0-R₂)ₙ-OR₃ (II)
in which R₁ is a hydrogen atom or a linear or branched C1 to C5 alkyl chain, R₂ is a linear or branched C1 to C5 alkyl chain, R₃ is a hydrogen atom or a linear or branched C1 to C5 alkyl chain and n is an integer from 1 to 200,000.

7. Compositions according to claim 6, **characterised in that** R₁ is a linear C1 to C3 alkyl chain and n assumes a value between 1 and 3.

8. Compositions according to either one of claims 6 and 7, **characterised in that** the proportion of liquefying solvent is from 0.5 to 50 % and preferably from 1 to 10 %.

9. Compositions according to any one of claims 1 to 8, **characterised in that** they are formulated in any pharmaceutical presentation used in cosmetics, medicated skin care or in veterinary applications for topical application for disinfecting or antimicrobial purposes: aqueous solutions, O/W and W/O emulsions, milks, lotions, gels, ointments, hair lotions, shampoos and conditioners, soaps, sticks, sprays, masks, poultices, dressings.

10. Compositions according to any one of claims 1 to 9, **characterised in that** they are capable of combination with any other ingredient used conventionally respectively in the fields of cosmetics, medicated skin care or veterinary medicine: natural or synthetic lipids, gelling, thickening, surface-active and emulsifying polymers, water-or fat-soluble active principles, plant extracts, tissue extracts, marine extracts, polyols, softeners, other antimicrobial agents.

11. Compositions according to any one of claims 1 to 10, **characterised in that** they have added thereto any useful or advantageous substances used conventionally in the field of the prevention of mastitis in milk-yielding animals by soaking, spraying or massage of the udders: iodine, iodophors, chloramine-T, hydrogen peroxide, or other bactericidal substances, as well as any emollient and moisturising substance (paraffin, synthetic or vegetable oils), any healing substance (allantoin, bisabolol, shea butter) and any therapeutic substance (plant extracts).

12. Use of the compositions according to claims 1 to 10 to obtain hydrogels intended for treatment and care of the skin, hair, nails and scalp, that is to say in particular treatment of acne, dandruff, impetigo and fungal infections and for deodorant treatment.

13. Use of the compositions according to claims 1 to 11 to obtain hydrogels intended for treatment and prevention of mastitis by topical application on the teats and udders of milk-yielding animals, for the decrease of the risk of transmission of Listeria to milk, and for the improvement of the condition of the skin of the teat.

14. Use of alkane-diol of the general formula R-CHOH-CH₂OH where R is a linear or branched alkyl chain of 3 to 12 carbon atoms, preferably a linear alkyl chain of 6 to 8 carbon atoms, to obtain hydrogels without glyceryl poly(meth)acrylate gel, intended for treatment of the skin, hair, nails and scalp, that is to say in particular treatment of acne, dandruff, impetigo, and fungal infections and for deodorant treatment.

15. Use of alkane-diol of the general formula R-CHOH-CH₂OH where R is a linear or branched alkyl chain of 3 to 12 carbon atoms, preferably a linear alkyl chain of 6 to 8 carbon atoms, to obtain hydrogels based on alkane-diol without glyceryl poly(meth)acrylate gel, intended for treatment and prevention of mastitis by topical application on the teats and udders of milk-yielding animals, for the decrease of the risk of transmission of Listeria to milk, and for the improvement of the condition of the skin of the teat.
